Europäisches Patentamt

**European Patent Office**

Office européen des brevets

(19)

(11) Veröffentlichungsnummer: **0 049 860**
**B1**

(12) **EUROPÄISCHE PATENTSCHRIFT**

(45) Veröffentlichungstag der Patentschrift:
03.10.84

(21) Anmeldenummer: **81108017.5**

(22) Anmeldetag: **06.10.81**

(51) Int. Cl.³: **C 07 C 103/46,** C 07 C 102/04,
C 07 D 207/452, G 01 N 33/78

(54) Verfahren zur Herstellung von reaktiven, kupplungsfähigen Derivaten der Schilddrüsenhormone T3 und T4 und deren Verwendung.

(30) Priorität: **07.10.80 DE 3037858**

(43) Veröffentlichungstag der Anmeldung:
**21.04.82 Patentblatt 82/16**

(45) Bekanntmachung des Hinweises auf die Patenterteilung:
**03.10.84 Patentblatt 84/40**

(84) Benannte Vertragsstaaten:
**AT BE CH DE FR GB IT LI LU NL SE**

(56) Entgegenhaltungen:
DE - A - 2 656 155
DE - A - 2 737 802
DE - C - 819 093
US - A - 2 803 654

ANGEWANDTE CHEMIE, 77. Jahrgang, 1965, Nr. 9,
Seiten 414-426 Weinheim, DE. L. BIRKOFER et al.: "Die Silylierung als Hilfsmittel in der organischen Synthese"
CHEMICAL ABSTRACTS, Band 87, Nr. 19, 7. November 1977, Seite 191, Nr. 147469q Columbus, Ohio, U.S.A. SHEUE-YANN CHENG et al.: "Affinity labeling of human serum prealbumin with N-Bromoacetyl-L-thyroxine"
CHEMICAL ABSTRACTS, Band 82, Nr. 3, 20. Januar 1975, Seite 214, Nr. 13313x Columbus, Ohio, U.S.A. R. HOFFENBERG et al.: "Application of mass spectrometry to the study of thyroxine and related compounds in biological fluids"
CHEMICAL ABSTRACTS, Band 85, Nr. 9, 30. August 1976, Seiten 591,592 Nr. 63313e Columbus, Ohio, U.S.A. A. LAWSON et al.: "Mass spectrometric studies of

(73) Patentinhaber: **BOEHRINGER MANNHEIM GMBH,**
**Patentabteilung, Abt. E Sandhofer**
**Strasse 112-132 Postfach 31 01 20,**
**D-6800 Mannheim 31 Waldhof (DE)**

(72) Erfinder: **Batz, Hans-Georg, Dr., Traubinger Strasse 63,**
**D-8132 Tutzing (DE)**
Erfinder: **Albert, Winfried, Dr., Moosstrasse 10,**
**D-8121 Pähl (DE)**
Erfinder: **Lenz, Helmut, Dr., Waldschmidtstrasse 7,**
**D-8132 Tutzing (DE)**
Erfinder: **Linke, Hans-Ralf, Dr., Andechserstrasse 7,**
**D-8919 Raisting (DE)**
Erfinder: **Stähler, Fritz, Dr., Heimgartenstrasse 4,**
**D-8132 Tutzing (DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel**
**Postfach 860820, D-8000 München 86 (DE)**

(56) Entgegenhaltungen: (Fortsetzung)
thyroxine and related compounds. Trimethylsilyl derivatives"
CHEMICAL ABSTRACTS, Band 94, Nr. 17, 27. April 1981, Seite 378, Nr. 135408a Columbus, Ohio, U.S.A. V.M. NIKODEM et al.: "Affinity labeling of rat liver thyroid hormone nuclear receptor"

Die Akte enthält technische Angaben, die nach dem Eingang der Anmeldung eingereicht wurden und die nicht in dieser Patentschrift enthalten sind.

## Beschreibung

Gegenstand der Erfindung ist ein Verfahren zur Herstellung von reaktiven, kupplungsfähigen Derivaten der Schilddrüsenhormone 3,3',5-Trijodthyronin ($T_3$) udn 3,3',5,5'-Tetrajodthyronin ($T_4$) durch Umsetzung mit reaktiven Carbonsäure-Derivaten sowie die Verwendung dieser reaktiven, kupplungsfähigen Derivate.

Die Bestimmung der Schilddrüsenhormone $T_3$ und $T_4$ im Serum hat für die klinische Diagnostik erhebliche Bedeutung. Üblicherweise werden heute zur Bestimmung immunologische Verfahren angewendet. Neben den radio-immunologischen Verfahren gewinnen heute enzym-immunologische Methoden zunehmend an Bedeutung.

Zur Herstellung von Antikörpern bindet man das Hormon ($T_3$, $T_4$), welches immunologisch als Hapten anzusehen ist, zunächst an ein hochmolekulares Protein, z.B. Albumin oder Polylysin. Man erhält so das eigentliche Antigen (Immunogen). Gegen diese Immunogene werden in den damit immunisierten Tieren Antikörperpopulationen gebildet, darunter auch spezifische Antikörper gegen das Hormon. Diese spezifischen Antikörper können durch Affinitätschromatographie abgetrennt werden, indem man die Antikörpermischung mit dem kovalent an ein Trägermaterial gebundenen Hormon behandelt.

Neben dem radio-immunologischen Nachweis mit radioaktiv markiertem Hormon gewinnt der enzym-immunologische Nachweis zunehmend an Bedeutung. Hier dient das Enzym bzw. die von ihm katalysierte Reaktion als Grundlage der Nachweismethode, wobei das Hormon kovalent an das Enzym gebunden sein muss.

Wie aus den geschilderten Anwendungen hervorgeht, besteht ein Bedarf an Derivaten der Schilddrüsenhormone $T_3$ und $T_4$, die eine spezifisch reagierende, funktionelle Gruppe aufweisen, welche unter schonendsten Bedingungen (pH-Wert von 6 bis 8, Raumtemperatur) mit funktionellen Gruppen von insbesondere Proteinen, wie Enzymen, reagieren kann. Als funktionelle Gruppen an Proteinen kommen vor allem die $NH_2$- und die SH-Gruppen in Frage. Dem Fachmann ist hier eine Reihe von spezifisch und schonend reagierenden Substanzen bekannt. So reagieren die Aminogruppen schon bei einem pH-Wert von 7 bis 8 mit reaktiven Estern, Imidoestern oder Glutardialdehyd. Mit SH-Gruppen reagieren spezifisch vor allem Halogenacetylgruppen, insbesondere Jodacetylgruppen, und Maleinimidogruppen.

Die Einführung von solchen spezifisch und empfindlich reagierenden Gruppen in andere Moleküle bereitet bei den Schilddrüsenhormonen $T_3$ und $T_4$ Schwierigkeiten, da diese in den meisten üblichen Lösungsmitteln unlöslich sind. Ein Auflösen ist nur in alkalischer wässriger Lösung möglich, wo bereits andere empfindlich reagierende Gruppen wieder hydrolysiert werden. Diese Schwierigkeiten werden meist dadurch umgangen, dass man statt der freien Schilddrüsenhormone $T_3$ oder $T_4$ die entsprechenden, an der Aminogruppe oder Carboxylgruppe geschützten Derivate einsetzt und über die dann noch jeweils freie zweite Funktion aktiviert und kuppelt. Auf die Abspaltung der Schutzgruppe wird in der Regel verzichtet, da hierbei wieder mit unerwünschten Nebenreaktionen zu rechnen ist.

Sehr klar werden diese Probleme am Beispiel eines Maleinimido-Derivats der Hormone $T_3$ bzw. $T_4$ zur SH-spezifischen Kupplung an Galactosidase. In der DE-OS 26 56 155 wird die Herstellung von o-Maleinimido-benzoyl-$T_3$ beschrieben. Bei dem Versuch, präparativ fassbare Mengen dieses Derivats herzustellen, wurden lediglich Ausbeuten von weniger als 5% erhalten, da das Schilddrüsenhormon $T_3$ in der Lösung nahezu unlöslich ist und die Maleinimido-Verbindung als zweite Reaktionskomponente schon nach wenigen Stunden der Reaktionszeit vollständig hydrolysiert, worauf in dieser DE-OS auch hingewiesen wird. Auf diese Instabilität haben auch E. Ishikawa et al. («Enzyme Labelled Immuno-Assay of Hormons and Drugs» (1978), Walter de Gruyter & Co., Berlin/New York) hingewiesen. Danach sind die N-Alkyl-maleinimido-Derivate wesentlich stabiler als die N-Benzoyl-Derivate. Aber selbst unter Anwendung solcher stabiler Derivate, wie etwa der 6-Maleinimidocapronsäure, konnte das entsprechende Derivat des Schilddrüsenhormons $T_4$ nur mit weniger als 5% Ausbeute erhalten werden (O. Keller und J. Rudinger, Helv. Chim. Acta 58 (1975) 531–541).

Aus EU-A- 6 998 ist die Kupplung von t-Butyloxycarbonylthyroxin-N-hydroxysuccinimid an Peroxidase bekannt. Dabei liegt $T_4$ in geschützter Form vor. Dies hat den Nachteil, dass seine immunologische Erkennbarkeit eingeschränkt ist. Weiterhin wird in Birkofer et al., Chem. Ber. 94 (1961) 1263 ff der Trimethylsilyl-Rest als Schutzgruppe für SH- und OH-Funktionen beschrieben. Eine Silylierung von Tyrosin wird übrigens nicht erwähnt.

Wohl aufgrund dieser Schwierigkeiten wurde auch schon vorgeschlagen, das m-Maleinimidobenzoyl-Derivat des $T_4$-Methylesters statt des freien Schilddrüsenhormons $T_4$ herzustellen (N. Monji et al., BBRC 85 (1978) 671–677). Auf eine Verseifung des Esters wird dann allerdings verzichtet.

Die Aufgabe der vorliegenden Erfindung besteht nun darin, die kostspieligen Schilddrüsenhormone $T_3$ und $T_4$ in wesentlich besserer Ausbeute als dies bisher möglich war, mit reaktionsfähigen Gruppen zu versehen, die dann die Kupplung mit einem polymeren Träger, namentlich einem Protein und insbesondere einem hochmolekularen Protein, ermöglichen.

Diese Aufgabe wird nun durch die kennzeichnenden Merkmale des Verfahrens gemäss Hauptanspruch gelöst.

Gegenstand der Erfindung ist daher ein Verfahren zur Herstellung von reaktiven, kupplungsfähigen Derivaten der Schilddrüsenhormone 3,3',5-Trijodthyronin ($T_3$) und 3,3',5,5'-Tetrajodthyronin ($T_4$) durch Umsetzung mit reaktiven Carbonsäurederivaten, das dadurch gekennzeichnet ist, dass man sämtliche funktionellen Gruppen des Schilddrüsenhormons durch Umsetzung mit einem reaktiven Triorganosilyl-Derivat silyliert und das erhaltene persilylierte Derivat des Schilddrüsenhormons mit einem an der Carboxylgruppe aktivierten bifunktionellen Carbonsäure-Derivat umsetzt. Die abhängigen Ansprüche 2–5 betreffen besonders bevorzugte Ausführungsformen dieses erfindungsgemässen Verfahrens. Gegenstand der Erfindung ist auch die Verwendung der mit Hilfe des erfindungsgemässen Verfahrens hergestellten reaktiven, kupplungsfähigen Derivate der genannten Schilddrüsenhormone $T_3$ und $T_4$ zur Herstellung von Kupplungsprodukten dieser Schilddrüsenhormone mit polymeren Trägern, insbesondere Proteinen, namentlich Enzymen, wie insbesondere β-Galactosidase.

Die erfindungswesentliche Massnahme des beanspruchten Verfahrens ist in der Persilylierung der Schilddrüsenhormone $T_3$ und $T_4$ zu sehen, bei der sämtliche funktionellen Gruppen mit dem Silylierungsmittel umgesetzt werden, was zur Folge hat, dass dieses Zwischenprodukt in vielen organischen Lösungsmitteln löslich wird und ein Zwischenprodukt anfällt, das ohne weiteres bei schonenden Bedingungen mit reaktiven Carbonsäure-Derivaten, wie Säurechloriden, Säureanhydriden und reaktiven Estern, umgesetzt werden kann.

Es ist bereits bekannt gewesen, dass sich Aminosäuren nach vollständiger Silylierung sehr leicht mit reaktiven Carbonsäure-Derivaten umsetzen lassen (L. Birkhofer et al., Angewandte Chemie 77 (1965) 414–426). Allerdings hat sich dieses Verfahren bei der Peptidsynthese nicht bewährt, da die üblicherweise eingesetzte Trimethylsilyl-Schutzgruppe sehr stark hydrolyseempfindlich ist und bereits durch die Einwirkung von Methanol abgespalten wird. Es hat sich jedoch gezeigt, dass diese Reaktivität erfindungsgemäss von grossem Vorteil ist, da bei der Abspaltung der Silylgruppe und namentlich der Trimethylsilylgruppe, andere empfindliche Gruppen, wie der Maleinimidorest, nicht angegriffen werden.

Als überraschend hat sich dabei herausgestellt, dass die erfindungsgemäss als Zwischenprodukte gebildeten persilylierten Schilddrüsenhormone $T_3$ und $T_4$ sich wesentlich leichter und mit besseren Ausbeuten umsetzen lassen als die entsprechende analoge einfache Aminosäure, nämlich Tyrosin.

Das erfindungsgemässe Verfahren besteht nun darin, dass man sämtliche funktionellen Gruppen des Schilddrüsenhormons $T_3$ (3,3',5-Trijodthyronin) bzw. $T_4$ (3,3',5,5'-Tetrajodthyronin) durch Umsetzung mit einem reaktiven Triorganosilyl-Derivat silyliert und dann das erhaltene persilylierte Derivat mit einem aktivierten Carbonsäure-Derivat umsetzt.

Gemäss einer bevorzugten Ausführungsform des erfindungsgemässen Verfahrens verwendet man als Triorganosilyl-Derivat ein reaktives Trialkylsilyl-Derivat, wie Hexamethyldisilazan, Trimethylchlorsilan und/oder Triäthylaminosilan.

Man bewirkt die Persilylierung vorzugsweise unter Anwendung einer äquimolaren oder überschüssigen Menge des Silylierungsmittels. Dabei kann man gegebenenfalls in Gegenwart eines unter den Reaktionsbedingungen inerten organischen Lösungsmittels arbeiten, wie eines polaren organischen Lösungsmittels, das keine Hydroxylgruppen oder nucleophile Substituenten aufweist, die selber silyliert werden können. Beispiele für solche Lösungsmittel sind aromatische Kohlenwasserstoffe, wie Benzol, Toluol, Xylol und dergleichen. Man kann die Reaktion auch in Gegenwart eines Katalysators und/oder eines Akzeptors für die im Verlaufe der Reaktion gebildete Säure durchführen, beispielsweise in Gegenwart von Schwefelsäure bei Anwendung von Hexamethyldisilazan oder in Gegenwart von Triäthylamin bei der Umsetzung mit Trimethylchlorsilan. Bei der Umsetzung arbeitet man bei einer Temperatur zwischen Raumtemperatur und der Siedetemperatur des Reaktionsgemisches bzw. des Lösungsmittels.

Bei dieser Silylierungsreaktion werden die Aminogruppen, die Hydroxylgruppen und die Carboxylgruppen silyliert, was zur Folge hat, dass die Hydroxylgruppen und die Carboxylgruppen geschützt werden, während die Aminogruppe des umgesetzten Schilddrüsenhormons $T_3$ bzw. $T_4$ aktiviert wird und damit für die Umsetzung mit einem aktivierten Carbonsäure-Derivat, wie einem Säurechlorid, einem Säureanhydrid oder einem reaktiven Ester, zur Verfügung steht.

In der zweiten Stufe des erfindungsgemässen Verfahrens wird das persilylierte Derivat des Schilddrüsenhormons mit einem aktivierten Carbonsäure-Derivat, namentlich einem an der Carboxylgruppe aktivierten, bifunktionellen Carbonsäure-Derivat, umgesetzt. Hierfür geeignete aktivierte Carbonsäure-Derivate sind reaktive Ester oder Imidoester oder reaktive Säurechloride oder Säureanhydride. Von den reaktiven Estern bzw. Imidoestern sind die Hydroxysuccinimidester bevorzugt. Erfindungsgemäss besonders bevorzugte aktivierte Carbonsäure-Derivate sind Acryloylchlorid, Acrylsäure-imidomethylester, Jodacetyl-p-nitrophenylester, Bromacetyl-hydroxysuccinimidester, Derivate der Carbobenzoxy-γ-aminobuttersäure, Carbobenzoxy-β-aminopropionsäure (die nach der Abspaltung des Carbobenzoxyrestes die Aminogruppe als zweite funktionelle Gruppe enthält) und/oder 6-Maleinimido-hexansäure-N-hydroxysuccinimidester. Weitere Beispiele für erfindungsgemäss geeignete reaktive Carbonsäure-Derivate finden sich in der DE-OS 26 31 656 und der DE-OS 22 37 083, wie beispielsweise Äthylenglykol-bis-propionsäure-bis-hydroxysuccinimidester, Oxy-bis-propionsäure-hydroxysuccinimidester, Oxysuccinimido-glutarsäure-aminoacetaldehyd-dimethylacetal, Methacryloyl-ω-hydroxycarbonsäure-oxysuccinimidester, Hydroxysuccinimido-N-2-hydroxyäthyl-N'-dimethylharnstoff-bernsteinsäureester, Acrylsäurehydroxysuccinimidester, Methacrylsäure-hydroxysuccinimidester, Acrylsäurehydroxybenzotriazolester, Methacrylsäurehydroxybenzotriazolester, Acrylsäure-2,4,5-trichlorphenylester, Methacrylsäure-2,4,5-trichlorphenylester, N-Vinyl-carbaminsäurehydroxysuccinimidester, N -Vinyl- carbaminsäure -1-hydroxybenzotriazolester, N- Vinyl - carbaminsäure,2,4,5 - trichlorphenylester, Polymere und Copolymere auf der Grundlage dieser ungesättigten Ester.

Nach der Kupplung mit dem reaktiven Carbonsäure-Derivat erhält man eine $T_3$- bzw. $T_4$-Verbindung, die ohne weiteres in wässrigem Medium mit einem Protein gekuppelt werden kann. Hierbei werden die übrigen Silylgruppen durch das wässrige Medium abgespalten, worauf die Reaktion der aktiven Gruppe im Falle der Maleinimido- und Halogenacetylgruppe zu einer Kupplung mit einer SH-Gruppe im Protein führt, im Falle der Biscarbonsäure und Aldehydcarbonsäure zu einer Kupplung mit $NH_2$-Gruppen und im Falle der Aminobuttersäure zur Kupplung mit COOH-Gruppen.

Gegenstand der Erfindung ist daher auch die Verwendung der in dieser Weise hergestellten reaktiven kupplungsfähigen Derivate der Schilddrüsenhormone $T_3$ und $T_4$ zur Herstellung von Kupplungsprodukten dieser Schilddrüsenhormone mit polymeren Trägern, insbesondere Proteinen und namentlich enzymatisch aktiven Proteinen. Aufgrund der unter Verwendung dieser reaktiven, kupplungsfähigen Derivate der genannten Schilddrüsenhormone möglichen schonenden Umsetzung eignet sich diese Methode auch zur Kupplung dieser Schilddrüsenhormone an enzymatische aktive Proteine, die dann im Rahmen des ELISA-Tests eingesetzt werden können. Besonders geeignet ist wegen ihrer relativ hohen Anzahl an SH-Gruppen, die nicht sämtlich für die enzymatische Aktivität erforderlich sind, die β-Galactosidase.

Die folgenden Beispiele dienen der weiteren Erläuterung der Erfindung. Diese Beispiele betreffen namentlich die erfindungsgemässe Herstellung von reaktiven, kupplungsfähigen Derivaten über die trimethylsilylierten Zwischenprodukte. Neben dem Vergleich der Umsetzungen mit dem Schilddrüsenhormon $T_4$ und der analogen einfachen Aminosäure, Tyrosin, wurde auch die Umsetzung von verschieden aktivierten Carboxyl-Derivaten mit Tris-trimethylsilyl-Derivaten des Schilddrüsenhormons $T_4$ untersucht und verglichen. Danach sind die reaktiven Chloride den reaktiven Estern überlegen. So konnte N-Propionyl-tyrosin nur über das Propionylchlorid, nicht aber über Propionsäure-p-nitrophenylester bzw. Propionsäure-hydroxysuccinimidester hergestellt werden. Mit dem Propionsäureimidomethylester entsteht entsprechend die Amidiniumverbindung.

Da sich das Tris-trimethylsilyl-Derivat des Schilddrüsenhormons $T_4$ besser umsetzen lässt als das Tris-trimethylsilyl-Derivat des Tyrosins, gelingt hier die Umsetzung mit dem Propionsäure-hydroxysuccinimidester mit guten Ausbeuten. Die Umsetzung mit dem Propionsäure-p-nitrophenylester verläuft wesentlich schlechter.

Als reaktive kupplungsfähige Derivate der Schilddrüsenhormone $T_3$ bzw. $T_4$ wurden erfindungsgemäss hergestellt: N-Acryloyl-thyroxin, N-Bromoacetyl-thyroxin, Carbobenzoxy-γ-aminobuttersäure-thyroxin, N-(6-Maleinimido-hexanoyl)-thyroxin sowie N-(6-Maleinimido-hexanoyl)-$T_3$.

Beispiel 1
Herstellung der silylierten Zwischenprodukte
A. Silyliertes Schilddrüsenhormon $T_4$ (silyliertes Tetrajodthyronin)

Man trocknet das Natriumsalz des Thyroxins während 12 Stunden über Phosphorpentoxid im Ölpumpenvakuum. Dann erhitzt man 799 mg (1 mMol) dieses getrockneten $T_4$-natriumsalzes mit 30 ml absolutem Chloroform, 550 mg (0,68 ml) Trimethylchlorsilan und 505 mg (0,70 ml, 5 mMol) Triäthylamin während 1,5 bis 2 Stunden unter Lichtausschluss zum Sieden am Rückfluss. Man erhält eine völlig klare, leicht gelbliche Lösung.

Die in dieser Weise hergestellten Ansätze von silyliertem $T_4$ werden stets sofort weiterverarbeitet und zwar soweit als möglich lichtgeschützt.

B. Silyliertes Schilddrüsenhormon $T_3$

Man wiederholt die Verfahrensweise, wie sie oben unter A. beschrieben wurde, unter Verwendung von 673 mg 3,3',5-Trijodthyronin anstelle des $T_4$.

C. Silyliertes Tyrosin

Man trocknet L-Tyrosin während 12 Stunden über Phosphorpentoxid im Ölpumpenvakuum. Dann erhitzt man 1,81 g (10 mMol) dieser getrockneten Aminosäure, 3,25 g (4,0 ml, 30mMol) Trimethylchlorsilan und 3,03 g (4,2 ml, 30 mMol) Triäthylamin in 50 ml absolutem Chloroform während 1 Stunde zum Sieden am Rückfluss. Bereits nach wenigen Minuten beginnt die Lösung klar zu werden. Der Ansatz wird stets sofort weiterverarbeitet.

Beispiel 2
N-Propionyl-tyrosin

Man lässt 10 mMol silyliertes Tyrosin (Beispiel 1, Stufe C.) während 20 Stunden bei 20 °C mit 0,925 g (10 mMol) Propionylchlorid reagieren, hydrolysiert dann mit 5 ml Methanol und engt mehrfach mit Methanol ein. Das Material wird mit Essigester über 500 ml Kieselgel (60) chromatographiert.
Ausbeute = 1,37 g (57% der Theorie)
Elementaranalyse:
berechnet: C 60,8%    H 6,37%    N 5,9%
gefunden:    61,8%       6,6%       5,47%
NMR-Sepktrum (in DDMSO): δ = 0,94 (t, $CH_3$), 6,83 (qu, $CH_{phenol}$).

Beispiel 3
N-Propionyl-tyrosin

Man wiederholt die Massnahmen von Beispiel 2, setzt jedoch anstelle von Propionylchlorid Propionsäure-hydroxysuccinimidester ein. Man erhält die Titelverbindung mit einer Ausbeute von 8%.

**Beispiel 4**
**N-Propionyl-tyrosin**
Man wiederholt die Massnahmen des Beispiels 2, verwendet jedoch anstelle von Propionylchlorid Propionsäure-p-nitrophenylester. Man erhält die Titelverbindung mit einer Ausbeute von 5%.

**Beispiel 5**
Man wiederholt die Massnahmen des Beispiels 2 unter Verwendung von Propionsäureimidomethylester anstelle des Propionylchlorids. Man erhält 1,1 g (46%) chromatographisch reines Propionylimidotyrosin (Amidin) in Form eines gelblichen Öls.
$R_f$-Wert: 0,42 (SiF-Platte, Laufmittel: Äthylacetat/Benzol/Eisessig/Wasser-Mischung 10/10/2/1).

**Beispiel 6**
**N-Propionyl-$T_4$**
Man rührt 0,5 mMol $T_4$ über Nacht bei Raumtemperatur mit 1,71 mg (1 mMol) Propionsäurehydroxysuccinimidester und hydrolysiert dann mit 1 ml Methanol und schliesslich mit Wasser. Man engt die entstandene Lösung ein, trocknet und extrahiert mit 40 ml Chloroform. Man engt den Extrakt erneut ein, extrahiert mit Aceton und kristallisiert den Rückstand des Acetonextrakts zweimal aus einer Methanol/Wasser-Mischung um.
Ausbeute = 188 mg (45% der Theorie).
Elementaranalyse:
berechnet: C 25,9%    H 1,81%    N 1,68%
gefunden:   25,5%      1,65%      1,60%
NMR-Spektrum (DDMSO): $\delta$ = 0,94 (t, $CH_3$), 7,06 (s, $CH_{Phenol}$), 7,8 (s, $CH_{Phenylen}$).

**Beispiel 7**
Man wiederholt die Massnahmen des Beispiels 6, verwendet jedoch anstelle von Propionsäurehydroxysuccinimidester den Propionsäure-p-nitrophenylester. Man erhält N-Propionyl- $T_4$ mit einer Ausbeute von 12%.

**Beispiel 8**
**N-Acryloyl-$T_4$**
Man rührt 2 mMol silyliertes $T_4$ (siehe Beispiel 1, A.) und 190 mg (2 mMol) frisch destilliertes Acryloylchlorid während 24 Stunden bei Raumtemperatur, hydrolysiert dann mit 5 ml Methanol, engt ein, behandelt mehrfach mit Methanol und engt erneut ein. Man nimmt den Rückstand mit Essigester auf und reinigt ihn über einer 250 ml Kieselgel-(60)-Säule chromatographisch.
Ausbeute = 130 mg (16% der Theorie)
Elementaranalyse:
berechnet: C 26,0%    H 1,58%    N 1,69%
gefunden:   27,7%      1,88%      1,56%.
NMR-Spektrum (DDMSO): $\delta$ = 5,7 (qu, CH=) 6,93 (s, $CH_{Phenol}$), 7,68 (s, $CH_{Phenylen}$).

**Beispiel 9**
**N-Bromacetyl-$T_4$**
Man stellt die Verbindung nach der Verfahrensweise von Beispiel 8 unter Verwendung von Bromacetyl-hydroxysuccinimidester anstelle von Acrylsäurechlorid her.

**Beispiel 10**
**N-Maleinimido-hexanoyl-$T_4$**
Man gibt 313 mg (1 mMol) 6-Maleinimido-hexansäure-N-hydroxysuccinimidester zu 1 mMol silyliertem $T_4$ (Beispiel 1, A.) in 40 ml absolutem Chloroform und rührt während 16 Stunden bei einer Badtemperatur von 60 °C. Anschliessend lässt man abkühlen, gibt 2,0 ml Methanol zu und rührt während 30 Minuten bei Raumtemperatur. Dann schüttelt man die Reaktionsmischung rasch mit 20 ml Wasser und 1 ml konzentrierter Essigsäure und dann noch zweimal mit jeweils 20 ml Wasser. Man trocknet mit Natriumsulfat und filtriert. Der Filterkuchen wird erneut mit 30 ml Chloroform extrahiert. Dann engt man die vereinigten Chloroformextrakte ein und kristallisiert aus dem öligen Rückstand über Nacht bei 4 °C die Substanz aus, die im Vakuum getrocknet wird.
Ausbeute = 300 mg (30% der Theorie)
Elementaranalyse:
berechnet: C 31,3%   H 2,29% N 2,97% J 52,8%
gefunden:    30,6%     2,19%    3,7%    50,5%
NMR-Spektrum (DDMSO): $\delta$ = 7,0 (s, CH=$_{Malein}$), 7,06 (s, $CH_{Phenol}$), 7,80 (s, $CH_{Phenylen}$).

**Beispiel 11**
**N-Maleinimido-hexanoyl-3,3',5-trijodthyronin**
Man wiederholt die Massnahmen des Beispiels 10, setzt jedoch anstelle von silyliertem $T_4$ silyliertes 3,3'-5-Trijodthyronin ($T_3$) ein.
Man erhält die Titelverbindung mit einer Ausbeute von 377 mg (32,8% der Theorie).

**Beispiel 12 (Anwendungsbeispiel)**
Bestimmung von $T_4$ im Humanserum mit Verwendung von β-Galactosidase-(6-maleinimido-hexanoyl)-thyroxin
Nach Beispiel 10 erhaltenes N-Maleinimido-hexanoyl-$T_4$ wird in wässriger Lösung mit β-Galactosidase gekuppelt. Das Kupplungsprodukt wird, wie nachstehend beschrieben, zur $T_4$-Bestimmung nach der ELISA-Methode verwendet:
Reagenzien: 0,01 ml hochaffines Kaninchen-Antithyroxin-Antiserum, dessen Verdünnung durch Titration mit dem $T_4$-Galactosidase-Konjugat bestimmt wurde, 0,02 ml β-Galactosidase-6-maleinimidohexanoyl- $T_4$-Konjugat in einer Verdünnung, die ohne Einsatz von unmarkiertem $T_4$ im Testsystem eine Extinktion von annähernd 1,0 A in 15 Minuten bei einer Wellenlänge 405 nm ergab,
0,02 ml $T_4$-Standard im Humanserum, Konzentration variiert zwischen 0 und 25 µg/100 ml,
0,23 ml Assay-Puffer (0,016 M Barbiturat-Puffer, pH 8,6, enthaltend 0,04% Tween 20).
Die Konjugat- und die Antiserumverdünnungen wurden mit Assay-Puffer hergestellt. Die obigen Reagenzien wurden zusammenpipettiert in ein Gefäss und 10 Minuten bei 22° inkubiert. In dieser Zeit konkurrierten die markierten und un-

markierten Formen des $T_4$ um Bindungsstellen am Antithyroxin-Antiserum.

Die Abtrennung des antikörpergebundenen und des freien $T_4$-$\beta$-Galactosidase-Konjugats erfolgte auf einem Immunadsorbens, bestehend aus einer Sepharose-4B-Säule, gekoppelt mit Kaninchen-Anti-$T_4$-Antikörpern, Volumen 1 ml. Das Gemisch wurde auf die Säule aufgetragen und mit 2 ml Assay-Puffer eluiert.

Das Eluat wurde mit 0,5 ml Substratlösung (0,1 M NaCl, 0,01 M MgCl$_2$, 0,1 M Mercaptoäthanol, 0,05 g/100 ml Natriumazid, 25 mM o-Nitrophenyl - $\beta$-D-Galactosid) versetzt und die Extinktion nach Ablauf von 15 Minuten Enzymreaktion photometrisch bei einer Wellenlänge von 405 nm gemessen.

Konzentration 0  2,5  5  12,5  25 µg/dl unmarkiertes $T_4$

Extinktion (15) 1,38 1,30 1,14 0,88 0,73 Säuleneluat

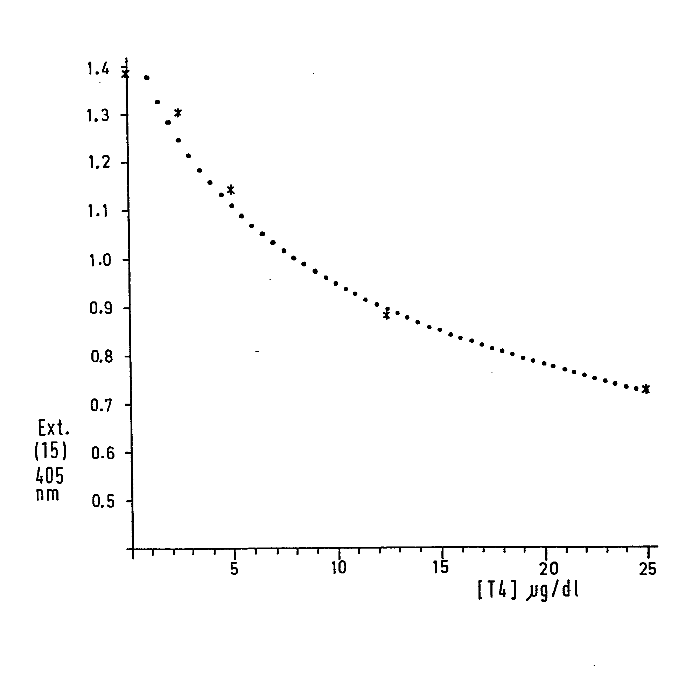

Die Abhängigkeit des Messsignals von der unmarkierten $T_4$-Konzentration lässt sich in der Form einer Bezugskurve ausdrücken, welche Fig. 1 der Zeichnung wiedergibt.

## Patentansprüche

1. Verfahren zur Herstellung von reaktiven, kupplungsfähigen Derivaten der Schilddrüsenhormone 3,3',5-Trijodthyronin ($T_3$) und 3,3',5,5'-Tetrajodthyronin ($T_4$) durch Umsetzung mit reaktiven Carbonsäurederivaten, dadurch gekennzeichnet, dass man sämtliche funktionellen Gruppen des Schilddrüsenhormons durch Umsetzung mit einem reaktiven Triorganosilyl-Derivat silyliert und das erhaltene persilylierte Derivat des Schilddrüsenhormons mit einem an der Carboxylgruppe aktivierten bifunktionellen Carbonsäure-Derivat umsetzt.

2. Verfahren nach Anspruch 1, dadurch gekennzeichnet, dass man als Triorganosilyl-Derivat ein reaktives Trialkylsilyl-Derivat verwendet.

3. Verfahren nach Anspruch 2, dadurch gekennzeichnet, dass man als reaktives Trialkylsilyl-Derivat Hexamethyldisilazan, Trimethylchlorsilan und/oder Triäthylaminosilan verwendet.

4. Verfahren nach den Ansprüchen 1 bis 3, dadurch gekennzeichnet, dass man die Persilylierung unter Anwendung einer äquimolaren oder überschüssigen Menge des Silylierungsmittels in Gegenwart eines unter den Reaktionsbedingungen inerten organischen Lösungsmittels und gegebenenfalls eines Katalysators bei einer Temperatur von Raumtemperatur bis zur Siedetemperatur des Lösungsmittels bewirkt.

5. Verfahren nach Anspruch 1, dadurch gekennzeichnet, dass man als aktiviertes Carbonsäure-Derivat einen reaktiven Ester oder Imidoester oder ein reaktives Säurechlorid oder Säureanhydrid einsetzt.

6. Verwendung der gemäss den Ansprüchen 1 bis 4 hergestellten reaktiven, kupplungsfähigen Derivate der Schilddrüsenhormone 3,3',5-Trijodthyronin ($T_3$) und 3,3',5,5'-Tetrajodthyronin ($T_4$) mit Propionylchlorid, Acryloylchlorid, Bromacetylhydroxysuccinimidester und/oder 6-Maleinimido-hexansäure-N-hydroxysuccinimidester zur Herstellung von unlöslichen Derivaten durch Kupplung mit polymeren Trägern oder Copolymerisation mit ungesättigten Monomeren.

## Revendications

1. Procédé de préparation de dérivés réactifs, couplables des hormones de la glande thyroïde 3,3',5-triiodothyronine ($T_3$) et 3,3',5,5'-tétraiodothyronine ($T_4$) par réaction avec des dérivés d'acide carboxylique réactifs, caractérisé en ce qu'on silyle la totalité des groupes fonctionnels de l'hormone de la glande thyroïde par réaction avec un dérivé triorganosilylé réactif, et en ce qu'on fait réagir le dérivé persilylé de l'hormone de la glande thyroïde obtenu avec un dérivé d'acide carboxylique bifonctionnel activé sur le groupe carboxyle.

2. Procédé suivant la revendication 1, caractérisé en ce qu'on utilise comme dérivé triorganosilylé un dérivé trialcoylsilylé réactif.

3. Procédé selon la revendication 2, caractérisé en ce qu'on utilise comme dérivé trialcoylsilylé réactif l'hexaméthyldisilazane, le triméthylchlorosilane et/ou le triéthylaminosilane.

4. Procédé suivant les revendications 1 à 3, caractérisé en ce qu'on effectue la persilylation en utilisant une quantité équimolaire ou en excès de l'agent de silylation en présence d'un solvant organique inerte dans les conditions réactionnelles et le cas échéant d'un catalyseur à une température allant de la température ambiante à la température d'ébullition du solvant.

5. Procédé selon la revendication 1, caractérisé en ce qu'on utilise comme dérivé d'acide carboxylique activé un ester ou imidoester réactif ou un chlorure d'acide ou un anhydride d'acide réactif.

6. Utilisation des dérivés réactifs, couplables des hormones de la glande thyroïde 3,3',5-triiodothyronine ($T_3$) et 3,3',5,5'-tétraiodothyronine ($T_4$) préparés conformément aux revendications 1 à 4, avec du chlorure de propionyle, du chlorure d'acryloyle, du bromacétylhydroxysuccinimidester et/ou du N-hydroxysuccinimidester de l'acide maléimido-hexanoïque pour la préparation de dérivés insolubles par couplage avec des supports polymères ou copolymérisation avec des monomères insaturés.

## Claims

1. Process for the preparation of reactive, couplable derivatives of the thyroid hormones 3,3',5-triiodothyronine ($T_3$) and 3,3',5,5'-tetraiodothyronine ($T_4$) by reaction with reactive carboxyl acid derivatives, characterised in that one silylates all functional groups of the thyroid hormone by reaction with a reactive triorganosilyl derivative and reacts the persilylated derivative of the thyroid hormone obtained with a fibunctional carboxylic acid derivative activated on the carboxyl group.

2. Process according to claim 1, characterised in that, as triorganosilyl derivative, one uses a reactive trialkylsilyl derivative.

3. Process according to claim 2, characterised in that, as trialkylsilyl derivative, one uses hexamethyldisilazane, trimethylchlorosilane and/or triethylaminosilane.

4. Process according to claims 1 to 3, characterised in that one brings about the persilylation with the use of an equimolar or excess amount of the silylation agent in the presence of an organic solvent inert under the reaction conditions and optionally of a catalyst at a temperature from room temperature to the boiling temperature of the solvent.

5. Process according to claim 1, characterised in that as activated carboxylic acid derivative, one uses a reactive ester or imido ester or a reactive acid chloride or acid anhydride.

6. The use of the reactive, couplable derivatives of the thyroid hormones 3,3',5-triiodothyronine ($T_3$) and 3,3',5,5'-tetraiodothyronine ($T_4$) prepared according to claims 1 to 4 with propionyl chloride, acryloyl chloride, bromoacetylhydroxy-succinimide ester and/or 6-maleinimido-hexanoic acid N-hydroxysuccinimide ester for the preparation of insoluble derivatives by coupling with polymeric carriers or co-polymerisation with unsaturated monomers.